# EUROPÄISCHE PATENTSCHRIFT

(11) **EP 3 330 960 B1**
(45) Veröffentlichungstag und Bekanntmachung des Hinweises auf die Patenterteilung: **17.11.2021**
(21) Anmeldenummer: 17204402.6
(22) Anmeldetag: 29.11.2017
(51) Int. Cl.: G09G 5/00, G06F 3/14, G09G 3/00, A61M 1/14, G06F 3/01

(54) **VORRICHTUNG ZUR EXTRAKORPORALEN BLUTBEHANDLUNG MIT EINER RUNDUM-ANZEIGE**
DEVICE FOR EXTRACORPOREAL BLOOD TREATMENT WITH AN ALL-ROUND DISPLAY
DISPOSITIF DE TRAITEMENT EXTRACORPOREL DU SANG DOTÉ D'UN AFFICHAGE PANORAMIQUE

(30) Priorität: 02.12.2016 DE 102016123371
(43) Veröffentlichungstag der Anmeldung: 06.06.2018
(73) Patentinhaber: B. Braun Avitum AG, 34212 Melsungen (DE)
(72) Erfinder: Brögger, Sebastian, 34593 Knüllwald (DE); Rieß, Armin, 34212 Melsungen (DE)
(74) Vertreter: Winter, Brandl - Partnerschaft mbB

(56) Entgegenhaltungen:
- EP-A1- 2 857 053
- JP-A- H09 107 514
- US-A1- 2007 180 129
- US-A1- 2015 002 490
- US-A1- 2016 152 370

## Beschreibung

Die Erfindung betrifft eine Vorrichtung zur extrakorporalen Blutbehandlung, beispielsweise eine Dialysemaschine, mit einer Personenortungs-/erkennungsvorrichtung, einer Steuereinheit und einer integrierten oder externen Anzeigevorrichtung, die dem Anwender Informationen von der Maschine anzeigt, beispielsweise Rückmeldungen zum Status des Therapieablaufs, der Wartung oder des Maschinenzustands und insbesondere eine Vorrichtung zur extrakorporalen Blutbehandlung gemäß dem Oberbegriff des Patentanspruchs 1.

### Hintergrund der Erfindung

Derzeit weisen Vorrichtungen zur extrakorporalen Blutbehandlung Anzeigevorrichtungen auf, die statisch oder dreh- und schwenkbar an dieser montiert sind und vom Anwender, wie z.B. medizinisches Fachpersonal, Techniker oder Patienten mit Heimdialyse, manuell mechanisch mit Bedienelementen wie Knöpfen oder über einen Touchscreen bedienbar sind. Dabei ist die Ausrichtung der Anzeige unabhängig von der räumlichen Position des Anwenders.

### Stand der Technik

Bei einer festen, statischen Anordnung des Anzeigebereichs gemäß einem bekannten Stand der Technik wird die Sichtbarkeit der angezeigten Informationen für den Anwender und dessen manueller Interaktionsbereich während des gewöhnlichen Betriebs stark eingeschränkt, da er sich zum Ablesen der Informationen und/oder zur Bedienung der Vorrichtung zur extrakorporalen Blutbehandlung direkt vor der fest installierten Anzeigevorrichtung befinden muss. Daher stellt die Einschränkung der Anzeigensichtbarkeit und des manuellen Interaktionsbereichs insbesondere für das Klinikpersonal, wie Ärzten und Pflegekräften, die im gewöhnlichen Klinikbetrieb mehrere Blutbehandlungsprozesse gleichzeitig betreuen, einen erheblichen Nachteil dar. Auch die Sichtbarkeit der angezeigten Information für den Patienten ist dadurch häufig nicht möglich. Auch für einen Servicetechniker, der die Vorrichtungen zur extrakorporalen Blutbehandlung wartet, ist der invariante manuelle Interaktionsbereich zur Bedienung der Vorrichtung zur extrakorporalen Blutbehandlung von Nachteil, da er das Bedienen mehrerer Maschinen gleichzeitig erschwert.

Vorrichtungen zur extrakorporalen Blutbehandlung mit einer dreh- und schwenkbaren Anzeigevorrichtung gemäß einem weiteren bekannten Stand der Technik verbessern die Handhabung eines bzw. mehrerer Maschinenabläufe insoweit, als dass die Anzeigenausrichtung manuell auf die Position des Anwenders ausgerichtet werden kann. Allerdings ist auch bei den Maschinen mit einer drehbaren/schwenkbaren Anzeigenvorrichtung ein Einwirken innerhalb des manuellen Interaktionsbereichs erforderlich und die Information können nur frontal vor der Anzeigenvorrichtung abgelesen werden. Eine dynamische Anpassung der Ausrichtung der Anzeigenvorrichtung auf die Position des Anwenders und eine Maschinen-Anwender-Interaktion außerhalb des manuellen Interaktionsbereichs ist nicht möglich.

Dies ist insbesondere in einem Fall problematisch, in dem mehrere Maschinen zur extrakorporalen Blutbehandlung gleichzeitig eine kritische Situation signalisieren, da der Anwender erst den jeweiligen Anzeigensichtbarkeitsbereich aller alarmierenden Maschinen betreten muss, um zu entscheiden, an welcher Maschine zuerst eingegriffen werden muss.

Aus dem Stand der Technik bekannt ist beispielsweise eine Dialysevorrichtung der Europäischen Patentanmeldung EP 2 857 053 A1 mit einer Bewegungserfassungseinrichtung und einer Gestensteuerung, mittels derer die Dialysevorrichtung angesteuert bzw. bedient werden kann.

Weiter offenbart die Patentanmeldung US 2015/0002490 A1 ein Ultraschallgerät mit mindestens einem Monitor, der abhängig von der Position und Blickrichtung des Bedieners so ausgerichtet wird, dass dieser die dargestellten Informationen gut einsehen kann.

In der Patentanmeldung US 2007/0180129 A1 ist ein medizinisches Gerät mit mehreren Monitoren offenbart, die abhängig vom Standort des Betrachters in ihrem Dreh-und/oder Neigungswinkel ausgerichtet werden können, sodass der Betrachter die Informationen gut einsehen kann.

### Kurze Beschreibung der Erfindung

Eine Aufgabe der vorliegenden Erfindung ist es daher, die vorgenannten Probleme der bekannten Anzeigenvorrichtungen an einer Vorrichtung zur extrakorporalen Blutbehandlung, zum Beispiel einer Dialysemaschine, zu lösen und eine Anzeigenvorrichtung zu konstruieren, die eine verbesserte Ablesbarkeit gewährleistet und die insbesondere dem Anwender, unabhängig von seiner relativen räumlichen Position zur Maschine, die Sichtbarkeit der angezeigten Informationen von der Maschine und eine Maschinen-Anwender-Interaktion außerhalb des manuellen Interaktionsbereichs, also des Bereichs, in dem der Anwender direkten manuellen Kontakt zur Bedienung der Maschine herstellen kann, ermöglicht.

Diese Aufgabe wird erfindungsgemäß durch die Merkmale des Anspruchs 1 gelöst. Vorteilhafte Weiterbildungen der Erfindung sind Gegenstand der beigefügten Unteransprüche.

Ein Grundgedanke der Erfindung besteht also darin, eine Vorrichtung zur extrakorporalen Blutbehandlung mit mindestens einer Anzeigevorrichtung und mindestens einer Personenortungs-/erkennungsvorrichtung bzw. -funktion derart auszustatten, dass die mindestens eine Anzeigevorrichtung die anzuzeigenden Informationen dynamisch zur Position des Anwenders/Person hin anzeigt, d.h. den Bewegungen des Anwenders/Person im Raum bzw. innerhalb eines definierten Umfelds um die Vorrichtung zur extrakorporalen Blutbehandlung herum nachführt, so dass sie (die Informationen) von der Position bzw. dem Standpunkt des Anwenders/Person aus auf der Anzeigevorrichtung einsehbar sind. Anders ausgedrückt orientiert sich die Anzeigerichtung für die anzuzeigenden Informationen am aktuellen Standpunkt und an der Bewegung des Anwenders im Raum bzw. innerhalb eines definierten Umfelds um die Vorrichtung zur extrakorporalen Blutbehandlung herum (Standpunktwechsel) und wird auf diesen (den Anwender) hin ausgerichtet und geführt. Darüber hinaus kann die Vorrichtung zur extrakorporalen Blutbehandlung optional mit einer weiteren Sensorik ausgestattet sein, die Informationen über die Gestik des Anwenders (Bewegungen des Anwenders am aktuellen Standpunkt im Raum bzw. innerhalb eines definierten Umfelds um die Vorrichtung zur extrakorporalen Blutbehandlung herum) einholt und verarbeitet und somit außerhalb des manuellen Interaktionsbereichs des Anwenders (Arm-Reichweite) durch dessen Steuerungsgesten in einem sensorischen Interaktionsbereich eine Maschinen-Anwender-Interaktion ermöglicht.

Konkreter ausgedrückt hat die Vorrichtung zur extrakorporalen Blutbehandlung die vorstehend genannte Anzeigevorrichtung, die Personenortungs-/erkennungsvorrichtung sowie eine Steuerung (Rechner), welche Signale der Personenortungs-/erkennungsvorrichtung empfängt und daraus der aktuellen Relativposition des Anwenders zur Vorrichtung zur extrakorporalen Blutbehandlung berechnet. Diese Berechnung kann zur Ermittlung des aktuellen Winkels (Himmelsausrichtung) zur Vorrichtung zur extrakorporalen Blutbehandlung und/oder der Distanz zur Vorrichtung zur extrakorporalen Blutbehandlung beinhalten. In Abhängigkeit der Berechnungsergebnisse steuert die Steuerung die Anzeigevorrichtung an, um deren Anzeigerichtung auf Position/Standpunkt des Anwenders hin auszurichten. Hierfür kann die Anzeigevorrichtung z.B. einen elektrisch angetriebenen Rotor aufweisen, der z.B. einen Monitor zumindest um eine Vertikalachse entsprechend dreht oder die Anzeigevorrichtung ist als Rundumanzeige ausgebildet, wobei die Informationsrichtung elektronisch auf der Rundumanzeige auf die Anwenderposition hin ausgerichtet wird.

Im Fall einer optionalen/zusätzlichen Distanzmessung kann in Abhängigkeit des Berechnungsergebnisses die Information groß- oder kleinformatig(er) dargestellt werden. Im Fall einer optionalen/zusätzlichen Gestik-Erkennungsfunktion können in Abhängigkeit erfasster/erkannter Bewegungen des Anwenders am bereits erfassten Standpunkt des Anwenders einzelne/ausgewählte Funktionen der Vorrichtung zur extrakorporalen Blutbehandlung aktiviert/deaktiviert/eingestellt steuerungsgestützt werden.

Vorteile einer Vorrichtung zur extrakorporalen Blutbehandlung mit einer solchen Anzeigevorrichtung und Personenortung/-erkennung liegen in der sofortigen, positionsunabhängigen Sichtung von angezeigten Informationen und der Möglichkeit optional zur sofortigen, (quasi ferngesteuerten) positionsunabhängigen Bedienung der Vorrichtung zur extrakorporalen Blutbehandlung (nachfolgend Dialysemaschine) durch den Anwender, sodass in einem Fall, in dem mehrere Maschinen gleichzeitig ein Alarmsignal abgeben, eine schnelle Priorisierung der Handlungen erfolgen kann. Somit ermöglicht die vorliegende Erfindung sowohl eine erhöhte Patientensicherheit im Alarmfall, als auch eine unkomplizierte Betreuung der Therapieabläufe an mehreren Maschinen gleichzeitig.

Die Aufgabe wird des Weiteren gelöst durch eine Vorrichtung zur extrakorporalen Blutbehandlung mit einer Steuereinheit (Steuerung), einer Anzeigevorrichtung und einer Personenortungs-/erkennungsvorrichtung. Die Personenortungs-/erkennungsvorrichtung ist dazu vorgesehen und angepasst, Informationen über den Standpunkt einer Person, die sich in einem Bereich befindet, der von der Personenortungs-/erkennungsvorrichtung erfassbar ist, einzuholen und zu verarbeiten. Die Steuereinheit empfängt die von der Personenortungs-/erkennungsvorrichtung eingeholten und verarbeiteten Informationen und die Personenortungs/- erkennungsvorrichtung sowie die Anzeigevorrichtung stehen über die Steuereinheit in Informationsaustauschkontakt zueinander. Erfindungsgemäß berechnet die Steuereinheit aus den empfangenen Informationen eine aktuelle Relativposition der Person zur Vorrichtung und die Anzeigevorrichtung ist mit einem außenliegenden, umlaufenden Display ausgestattet, das von sämtlichen Positionen innerhalb des von der personenortungs-/erkennungsvorrichtung erfassbaren Bereichs aus einsehbar ist, welches dazu angepasst ist, die Anzeigerichtung der anzuzeigenden Informationen in Abhängigkeit von der Relativposition der Person, insbesondere zur Vorrichtung zur extrakorporalen Blutbehandlung, bevorzugt automatisch so auf den Standpunkt der Person hin auszurichten, dass die anzuzeigenden Informationen vom Standpunkt der Person aus gesichtet werden können.

In anderen Worten ausgedrückt, erfasst die Personenortungs/-erkennungseinrichtung, die einer Vorrichtung zur extrakorporalen Blutbehandlung zugeordnet ist, eine Person in einem definierten Umfeld dieser Vorrichtung (entsprechend der Erfassungsleistung eines verwendeten Sensors) zur extrakorporalen Blutbehandlung und die Steuereinheit, die Bestandteil der Vorrichtung zur extrakorporalen Blutbehandlung ist, steuert die Anzeigevorrichtung, deren Display von sämtlichen Positionen innerhalb des definierten Umfeld aus einsehbar ist, so dass die Anzeige der darzustellenden Information automatisch zum Standpunkt der Person im definierten Umfeld hin erfolgt. Unter dem definierten Umfeld um die Vorrichtung zur extrakorporalen Blutbehandlung herum ist der ganze Bereich zu verstehen, der von der Sensorik der Personenerkennungsvorrichtung erfasst wird.

Vorteilhafterweise wird der Benutzer der Vorrichtung zur extrakorporalen Blutbehandlung, darunter sind Ärzte, Pflegepersonal, Techniker oder Patienten zu verstehen, bei einer solchen Ausgestaltung der Vorrichtung zur extrakorporalen Blutbehandlung als Person von der Personenortungs-/erkennungsvorrichtung erkannt und die auf dem Display der Anzeigevorrichtung dazustellenden Informationen werden automatisch in Richtung des Standpunkts der Person angezeigt und abhängig von ihrer Bewegung mitgeführt, sodass der Anwender sich unabhängig von der Anordnung des Displays an der Maschine im Therapieraum bewegen kann, ohne dabei den Sichtkontakt zu den dargestellten Informationen über den Therapieverlauf und/oder den Maschinenzustand zu verlieren. Eine solche Anzeigevorrichtung ist, beispielsweise zylinderartig oder polyedrisch, mit einem außenliegenden, umlaufenden Display ausgestattet, dabei ist auch eine Anordnung mehrerer Displayflächen zu einer Displaygesamtfläche möglich. An der Anzeigevorrichtung können auch Knöpfe zur manuellen Bedienung der Anzeigevorrichtung angebracht sein, auch die Integration eines Touchscreens zur Bedienung der Anzeige ist möglich. In einer weiteren Gestaltungsform der erfindungsgemäßen Anzeigevorrichtung sind mehrere Displays an verschiedenen Stellen des Raums so angebracht, dass von jedem Standpunkt im Raum mindestens ein Display einsehbar ist. Durch die dynamische Anpassung der Anzeigerichtung in Abhängigkeit des Standpunkts des Benutzers ist ein manuelles Eingreifen zur Justierung der Anzeigerichtung nicht nötig, wodurch auch eine Entfernung des Benutzers aus dem manuellen Interaktionsbereich keine Einschränkung für die sofortige Sichtung von dargestellten Informationen bedeutet. Der Benutzer ist damit in der Lage, im Umfeld der Vorrichtung zur extrakorporalen Blutbehandlung weitere Arbeiten, z.B. die Bedienung einer weiteren Maschine, auszuführen, ohne dabei die Kontrolle über eine sichere Durchführung des Therapieablaufs an der Vorrichtung zur extrakorporalen Blutbehandlung einzuschränken.

Bevorzugt ist die Personenortungs-/erkennungsvorrichtung dazu vorgesehen und angepasst, Informationen über Gesten der Person einzuholen und zu verarbeiten, und die Anzeigevorrichtung ist dazu angepasst, durch die Informationen über die Gesten der Person bedienbar zu sein.

Alternativ dazu hat die Personenortungs-/erkennungsvorrichtung eine gesonderte Sensorik, die dazu vorgesehen und angepasst ist, Informationen über Gesten der Person einzuholen und zu verarbeiten, und die Anzeigevorrichtung ist dazu angepasst, durch die Informationen über die Gesten der Person bedienbar zu sein.

Die Bedienung der Anzeigevorrichtung durch Gesten ermöglicht dem Benutzer die Bedienung des Displays außerhalb des manuellen Interaktionsbereichs. Somit kann er für eine schnelle Anpassung der Darstellung sorgen, z.B. Schriftgröße oder Umschalten des Informationsmenüs, ohne seinen Standpunkt im Raum ändern zu müssen. Eine Bedienungsmöglichkeit der Dialysemaschine durch Gestik außerhalb des manuellen Interaktionsbereich bedeutet für den Benutzer eine erhebliche Erleichterung, da er auch aus einer Entfernung, in der ein sofortiger manueller Eingriff nicht möglich ist, reagieren kann. Insbesondere in Situationen, in denen ein kritischer Alarm an einer Maschine gemeldet wird, dient eine sofortige Reaktionsmöglichkeit der Sicherheit des Patienten. Darüber hinaus ermöglicht ein sensorischer Interaktionsbereich, der über den manuellen Interaktionsbereich hinausreicht, dem Benutzer eine übersichtlichere Handhabung mehrerer Therapieprozesse parallel.

Weiter bevorzugt ist die Vorrichtung zur extrakorporalen Blutbehandlung mit Benutzerprofilen betreibbar, die jeweils einer Benutzeridentität zugeordnet sind, und die Personenerkennungsvorrichtung ist angepasst, Informationen über eine Benutzeridentität der Person einzuholen und zu verarbeiten, und die Anzeigevorrichtung ist dazu vorgesehen und angepasst, auf einem Display Informationen in Abhängigkeit von der Benutzeridentität der Person gemäß dem zugehörigen Benutzerprofil darzustellen, und die Anzeigevorrichtung und/oder Dialysemaschine ist/sind dazu vorgesehen und angepasst, in Abhängigkeit von der Benutzeridentität der Person gemäß dem zugehörigen Benutzerprofil bedienbar zu sein.

Bei einer solchen Ausgestaltung ist es im Betrieb der Vorrichtung zur extrakorporalen Blutbehandlung möglich, einzelnen Benutzern persönliche Anzeigeeinstellungen, z.B. Größe, Helligkeit, optische Sortierung von Informationsgruppen u.a., und benutzerabhängige Berechtigungen zur Bedienung der Maschine automatisch zuzuweisen. Im Regelfall werden sich die Berechtigungen zur Vornahme bestimmter Änderungen und Festlegungen, z.B. Erstellen eines Therapieablaufs, Ändern eines Therapielaufs, oder Eingreifen in einen laufenden Therapieablauf, zwischen Benutzergruppen wie Ärzten, Pflegepersonal und Technikern unterscheiden. Durch die benutzerabhängige Übernahme von Darstellungseinstellungen wird der Bedienkomfort des Benutzers erhöht. Mit einer Personenerkennungsvorrichtung, die in der Lage ist, eine Identitätsfeststellung der Person im sensorischen Interaktionsbereich der Maschine durchzuführen, kann außerdem sichergestellt werden, dass ausschließlich Bedienfunktionen ausgeführt werden können, für die der Benutzer autorisiert ist. Eine Situation, in der ein erster Benutzer an der Maschine angemeldet ist und ein zweiter Benutzer aus dem Umkreis der Maschine eine Bedienung mit den Berechtigungen des ersten Benutzers vornimmt, wird mit einer solchen Benutzeridentitätserkennung verhindert. Dies sichert den Zugriff auf den Therapieablauf gegen Unbefugte ab und stellt eine weitere Maßnahme zur Erhöhung der Patientensicherheit dar. Eine einfache Möglichkeit zur Identitätsfeststellung bietet das Auslesen eines Chips, der z.B. in mitgeführten Dienstausweisen des Klinikpersonals oder im Gastausweis oder in der Patientenchipkarte des Patienten integriert ist und von der Personenerkennungsvorrichtung ausgelesen werden kann.

Außerdem bevorzugt ist, dass die Personenerkennungsvorrichtung wenigstens eine Kamera aufweist. Eine Kamera bietet eine gut bekannte, kostengünstige Sensorik, mit der eine Personenerkennung einfach möglich gemacht werden kann. Besonders bevorzugt ist, dass die Personenerkennungsvorrichtung eine Gesichtserkennungsvorrichtung aufweist. Eine Gesichtserkennung bietet eine weitere Möglichkeit zur Identitätsfeststellung eines Benutzers im Umkreis der Vorrichtung zur extrakorporalen Blutbehandlung und ersetzt das Mitführen eines Dienstausweises zur Bedienung der Maschine. Darüber hinaus ist die Gesichtserkennung einer mitgeführten Chipkarte insofern überlegen, als ein Vertauschen von Dienstausweisen dazu führen kann, dass ein Benutzer im Besitz einer fremden Chipkarte mit Berechtigungen an der Maschine agiert, für die er nicht autorisiert ist. Auch die Gefahr, dass ein Dienstausweis unbemerkt verloren geht und eine Bedienung der Maschine dadurch blockiert wird, wird mit einer Gesichtserkennung ausgeräumt.

Bevorzugt ist die Vorrichtung zur extrakorporalen Blutbehandlung mit einer Vorrichtung für einen akustischen Alarm, zum Beispiel eine Sirene, ausgestattet und die Personenerkennungsvorrichtung und die Vorrichtung für einen akustischen Alarm stehen über die Steuereinheit in Informationsaustauschkontakt zueinander und die Vorrichtung für einen akustischen Alarm ist vorgesehen und angepasst, durch Signale der Personenerkennungsvorrichtung bedienbar zu sein.

Durch einen akustischen Alarm an der Vorrichtung zur extrakorporalen Blutbehandlung wird sichergestellt, dass auch ein Benutzer außerhalb des Sichtbereichs oder ein der Anzeige abgewandter Benutzer alarmiert werden kann. Dies ist insbesondere bei hochkritischen Fehlern vorteilhaft, um eine schnelle Reaktion durch das Klinikpersonal zu befördern. Durch die Verbindung von der Vorrichtung für den akustischen Alarm und der Personenerkennungsvorrichtung kann der Alarm automatisch inaktiviert werden, wenn der autorisierte Benutzer durch Gestik in den Therapieprozess eingreift.

Außerdem bevorzugt ist, dass die Anzeigevorrichtung ein Display mit einer konvex gebogenen, dem Krümmungsmittelpunkt abgewandten Displayfläche aufweist. Ein Display solcher Form kann vom Benutzer von mehreren Standpositionen im Raum aus eingesehen werden, abhängig vom Krümmungsradius und Umfang ist ein Display solcher Form von allen Standpunkten im Raum aus einsehbar. Somit kann eine Informationsdarstellung auf dem gewölbten Display stets zum Standpunkt des Anwenders hin automatisch angezeigt werden, sodass die Informationen stets vom Anwender einsehbar sind, ohne dass sein aktives Eingreifen erforderlich ist. Die Krümmung der Displayfläche muss dabei nicht unbedingt 360° umschließen, auch eine Öffnung zur hinteren Seite der Maschine hin ist vorteilhaft, da sich eine Maschine mit einer solchen Anzeigevorrichtung in einen kleinen Raum wandständig gut integrieren lässt.

Bevorzugt ist die Anzeigevorrichtung dazu angepasst, im Falle eines Alarms eine Kritikalitätsbewertung anzuzeigen. Dies erlaubt dem Benutzer eine sofortige Entscheidung über die Handlungsdringlichkeit, insbesondere im Fall von Alarmen an mehreren Maschinen gleichzeitig.

Weiter bevorzugt ist ein Netzwerk aus mehreren Vorrichtungen zur extrakorporalen Blutbehandlung mit den beschriebenen Merkmalen so angepasst, dass die Anzeigevorrichtungen der Vorrichtungen zur extrakorporalen Blutbehandlung, im Fall von parallelen Alarmen an mehreren Maschinen die auf den Displayflächen der Anzeigevorrichtungen darzustellenden Informationen gemäß der Priorität der Alarme darzustellen.

Eine automatisierte Priorisierung von parallelen Alarmen macht den Benutzer zur sofortigen Fehlerbehebung handlungsbereit, da die vorangehende notwendige Dringlichkeitsanalyse der parallel gemeldeten Fehler in einem solchen Netzwerk entfällt. Somit kann gewährleistet werden, dass ein kritischer Fehler in minimaler Zeit behoben und die Patientensicherheit weiter erhöht wird.

### Kurzbeschreibung der Zeichnungen

Nachfolgend werden bevorzugte Ausführungsbeispiele der Erfindung anhand der beigefügten Zeichnungen näher beschrieben.
Fig. 1 zeigt eine vereinfachte Darstellung einer erfindungsgemäßen Vorrichtung zur extrakorporalen Blutbehandlung mit einer Anzeigevorrichtung, die ein außenliegendes konvex gewölbtes Display ausweist, und einer Personenerkennungsvorrichtung.
Fig. 2 zeigt eine schematische Darstellung der Interaktionsbereiche um die Vorrichtung zur extrakorporalen Blutbehandlung herum.
Fig. 3 zeigt eine vereinfachte Draufsicht einer Anzeigevorrichtung mit polyedrischen Display und mehreren Displayteilflächen mit jeweils einem Sichtwinkel a.
Fig. 4 zeigt eine schematische Darstellung der Vorrichtung zur extrakorporalen Blutbehandlung mit erfindungsgemäßen Maschinenkomponenten.

### Detaillierte Beschreibung bevorzugter Ausführungsbeispiele

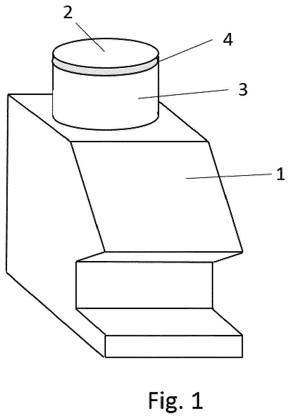

Fig. 1 zeigt eine vereinfachte Darstellung einer Vorrichtung zur extrakorporalen Blutbehandlung 1 mit einer Anzeigevorrichtung 2, die im vorliegenden Fall ein außenliegendes konvex gewölbtes Display 3 ausweist, und einer Personenortungs-/erkennungsvorrichtung 4. Die Anzeigevorrichtung 2 ist bevorzugt im oberen Bereich der Vorrichtung zur extrakorporalen Blutbehandlung 1 (Dialysemaschine) so angeordnet, dass sie sich im Sichtbereich des Anwenders befindet. Die Fläche des gewölbten Displays 3 an der Anzeigevorrichtung 2 ist konvex gewölbt und dem Krümmungsmittelpunkt abgewandt, sodass sie dem Anwender im Umkreis (vorzugsweise 360°) der Vorrichtung zur extrakorporalen Blutbehandlung 1 ggf. von allen Standpunkten im Raum aus zugewandt ist. Im bevorzugt oberen Bereich der Anzeigevorrichtung 2 befindet sich die Personenortungs-/erkennungsvorrichtung 4, deren Sensorik den (kompletten) Umkreis der Vorrichtung zur extrakorporalen Blutbehandlung 1 erfasst. Der Standpunkt einer Person im Umkreis der Vorrichtung zur extrakorporalen Blutbehandlung 1 wird über die Personenortungs-/erkennungsvorrichtung 4 erfasst und die Erfassungssignale einer Steuerung/Steuereinheit/Rechner vorzugsweise der Vorrichtung zur extrakorporalen Blutbehandlung 1 zugeführt. Da Vorrichtungen zur extrakorporalen Blutbehandlung 1 bereits zum Stand der Technik zählen und diese bekannter Maßen immer mit einer Steuerungseinheit zur Steuerung/Regelung der vorrichtungsinternen Funktionen ausgerüstet sind, kann an dieser Stelle auf eine genauere Beschreibung eines solchen Vorrichtung sowie deren Steuerung verzichtet werden, stattdessen kann auf den einschlägigen Stand der Technik beispielsweise der Anmelderin dieser Anmeldung selbst verwiesen werden. Die Steuerung steuert hierauf die Anzeigevorrichtung 2 derart an, dass über deren gewölbtes Display die darzustellenden Informationen stets in Richtung des Anwenders angezeigt werden können.

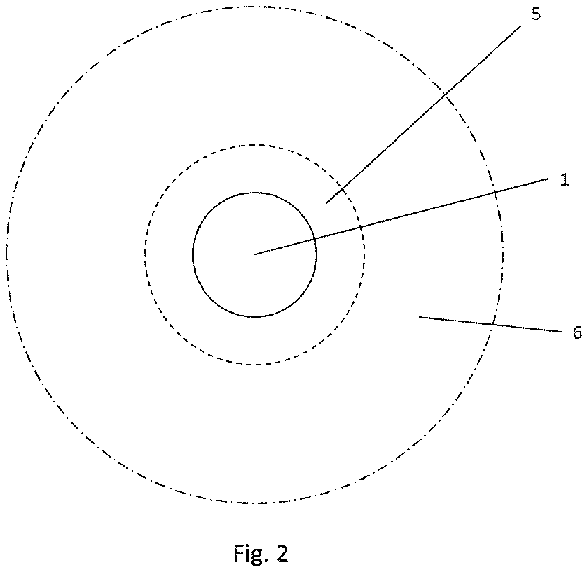

Fig. 2 zeigt eine schematische Darstellung der Interaktionsbereiche um die Vorrichtung zur extrakorporalen Blutbehandlung 1 herum. Der manuelle Interaktionsbereich 5 (durchschnittliche Armlänge) ist der durch die unterbrochene Linie angedeutete Bereich um die Vorrichtung zur extrakorporalen Blutbehandlung 1 herum, in dem der Anwender direkten Kontakt zur Vorrichtung zur extrakorporalen Blutbehandlung 1 herstellen kann, um beispielweise Betätigungselemente wie Knöpfe und Schalter an der Maschine oder der Anzeigevorrichtung 2 oder einen Touchscreen an der Anzeigevorrichtung 2 manuell zu bedienen. Von der Vorrichtung zur extrakorporalen Blutbehandlung 1 aus über den manuellen Interaktionsbereich hinaus erstreckt sich der sensorische Interaktionsbereich 6, der mit einer Strichpunktlinie angedeutet ist. Innerhalb des sensorischen Interaktionsbereichs 6 wird der Anwender von der Personenerkennungsvorrichtung 4 (in Fig. 2 nicht gezeigt) erkannt und die über die Anzeigevorrichtung 2 dargestellten Informationen werden in Richtung des Anwenders angezeigt. Innerhalb des sensorischen Interaktionsbereichs 6 können durch die Personenortungs-/erkennungsvorrichtung 4 z.B. Gesten und/oder die Benutzeridentität des Anwenders erfasst werden, sodass der Anwender die Vorrichtung zur extrakorporalen Blutbehandlung 1 und/oder Anzeigevorrichtung 2 innerhalb des sensorischen Interaktionsbereichs 6 berührungslos in bevorzugt ausgewählter/individuell auf den Anwender reduzierter Weise bedienen kann.

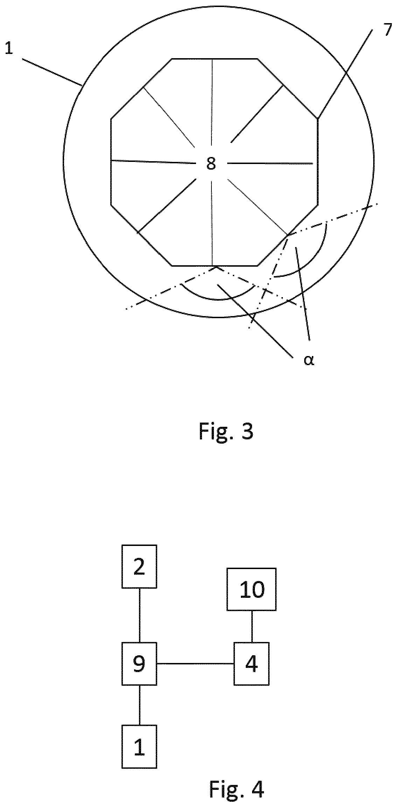

Fig. 3 zeigt eine vereinfachte Draufsicht einer Anzeigevorrichtung 2 mit einem polyedrischen Display 7 und mehreren Displayteilflächen 8 mit jeweils einem Sichtwinkel a. Die polyederartige Form wird durch ebene, aneinandergrenzende Displayteilflächen 8 realisiert, die dem Mittelpunkt des Polyeders abgewandt sind und in unterschiedliche Richtungen des Raums ausgerichtet sind. Der Sichtwinkel α ist so gewählt, dass von jedem Standpunkt aus innerhalb des Umkreises um die Vorrichtung zur extrakorporalen Blutbehandlung 1 herum mindestens eine Displayteilfläche 8 vom Anwender einsehbar ist. Zur einfacheren Ansicht wurde der Sichtwinkel α nur an einer Displayteilfläche 8 angedeutet.

Fig. 4 zeigt eine schematische Darstellung der Vorrichtung zur extrakorporalen Blutbehandlung mit erfindungsgemäßen Maschinenkomponenten. Die Vorrichtung zur extrakorporalen Blutbehandlung 1 ist mit einer Steuereinheit 9 ausgestattet, die mit der Anzeigevorrichtung 2 und der Personenortungs-/erkennungsvorrichtung 4 verbunden ist. Die Personenortungs-/erkennungsvorrichtung 4 ist bevorzugt mit einer Gesichtserkennung 10 ausgestattet. Signale, die von der Sensorik der Personenortungs-/erkennungsvorrichtung 4 erfasst/erzeugt werden, beispielsweise der Standort einer Person im Umfeld der Vorrichtung zur extrakorporalen Blutbehandlung 1, ggf. deren Gesten und/oder Benutzeridentität sowie ggf. Signale der verbundenen Gesichtserkennung 10, werden an die Steuereinheit 9 weitergeleitet. In Abhängigkeit von den Signalen der Personenortungs-/erkennungsvorrichtung 4 regelt die Steuereinheit 9 die Darstellung der Informationen auf Anzeigevorrichtung 2, beispielsweise wird in Abhängigkeit des von der Personenortungs-/erkennungsvorrichtung 4 erfassten aktuellen Standpunkts der Person im Umkreis der Vorrichtung zur extrakorporalen Blutbehandlung 1 die Anzeigerichtung der darzustellenden Informationen auf dem Display der Anzeigevorrichtung 2 geregelt. Ebenso werden Signale von der Vorrichtung zur extrakorporalen Blutbehandlung 1 an die Steuereinheit 9 weitergeleitet, die in Abhängigkeit von den übermittelten Informationen aus der Vorrichtung zur extrakorporalen Blutbehandlung 1 die von der Anzeigevorrichtung 2 darzustellenden Informationen regelt. Auch die benutzerabhängigen Berechtigungen für die Bedienung der Vorrichtung zur extrakorporalen Blutbehandlung 1 und der Anzeigevorrichtung 2 werden über die Steuereinheit 9 geregelt. Dazu wird die Benutzeridentität von der Personenerkennungsvorrichtung 4, beispielsweise durch Auslesen eines personenbezogenen Chips, bzw. durch Gesichtserkennung von der Gesichtserkennungsvorrichtung 10 erfasst und an die Steuereinheit 9 weitergeleitet, die in Abhängigkeit vom zugehörigen auf der Steuereinheit 9 eingespeicherten Benutzerprofil die Bedieneingaben des Anwenders zulässt oder blockiert.

### Bezugszeichenliste

- 1: Vorrichtung zur extrakorporalen Blutbehandlung
- 2: Anzeigevorrichtung
- 3: Gewölbtes Display
- 4: Personenortungs-/erkennungsvorrichtung
- 5: Manueller Interaktionsbereich
- 6: Sensorischer Interaktionsbereich
- 7: Polyedrisches Display
- 8: Displayteilfläche
- 9: Steuereinheit
- 10: Gesichtserkennungsvorrichtung

## Patentansprüche

1. Vorrichtung (1) zur extrakorporalen Blutbehandlung mit einer Steuereinheit (9), einer Anzeigevorrichtung (2) und einer Personenortungs-/erkennungsvorrichtung (4), wobei
die Personenortungs-/erkennungsvorrichtung (4) dazu vorgesehen und angepasst ist, Informationen zumindest über den Standpunkt einer Person, die sich in einem Bereich befindet, der von der Personenortungs-/erkennungsvorrichtung (4) erfassbar ist, einzuholen und zu verarbeiten,
die Steuereinheit (9) die von der Personenortungs-/erkennungsvorrichtung (4) eingeholten und verarbeiteten Informationen empfängt und
die Personenortungs-/erkennungsvorrichtung (4) und die Anzeigevorrichtung (2) über die Steuereinheit (9) in Informationsaustauschkontakt zueinander stehen, **dadurch gekennzeichnet, dass**
die Steuereinheit (9) aus den empfangen Informationen eine aktuelle Relativposition der Person zur Vorrichtung (1) berechnet,
die Anzeigevorrichtung (2) mit einem außenliegenden, umlaufenden Display (3; 7) ausgestattet ist, das von sämtlichen Positionen innerhalb des von der Personenortungs-/erkennungsvorrichtung (4) erfassbaren Bereichs aus einsehbar ist, und
die Anzeigevorrichtung (2) dazu vorgesehen und angepasst ist, die Anzeigerichtung der anzuzeigenden Informationen in Abhängigkeit zumindest von der Relativposition der Person zur Vorrichtung (1) zur extrakorporalen Blutbehandlung automatisch so auf den Standpunkt der Person hin auszurichten, dass die anzuzeigenden Informationen vom Standpunkt der Person aus gesichtet werden können.

2. Vorrichtung (1) zur extrakorporalen Blutbehandlung nach Anspruch 1, **dadurch gekennzeichnet, dass**
die Personenortungs-/erkennungsvorrichtung (4) dazu vorgesehen und angepasst ist, Informationen über Gesten der Person einzuholen und zu verarbeiten, und
die Anzeigevorrichtung (2) dazu angepasst ist, durch die Informationen über die Gesten der Person bedienbar zu sein.

3. Vorrichtung (1) zur extrakorporalen Blutbehandlung nach Anspruch 1 oder 2, **dadurch gekennzeichnet, dass**
die Personenortungs-/erkennungsvorrichtung Personenerkennungsvorrichtung (4) dazu angepasst ist, Informationen über Gesten der Person einzuholen und zu verarbeiten, und
die Vorrichtung (1) zur extrakorporalen Blutbehandlung dazu angepasst ist, durch die Informationen über die Gesten der Person bedienbar zu sein.

4. Vorrichtung (1) zur extrakorporalen Blutbehandlung nach Anspruch 1 bis 3, **dadurch gekennzeichnet, dass**
die Vorrichtung (1) zur extrakorporalen Blutbehandlung mit Benutzerprofilen, die jeweils einer Benutzeridentität zugeordnet sind, betreibbar ist und
die Personenortungs-/erkennungsvorrichtung (4) angepasst ist, Informationen über eine Benutzeridentität der Person einzuholen und zu verarbeiten, und
die Anzeigevorrichtung (2) dazu angepasst ist, auf einem Display (3; 7) Informationen in Abhängigkeit von der Benutzeridentität der Person gemäß dem zugehörigen Benutzerprofil darzustellen.

5. Vorrichtung (1) zur extrakorporalen Blutbehandlung nach Anspruch 1 bis 4, **dadurch gekennzeichnet, dass**
die Vorrichtung (1) zur extrakorporalen Blutbehandlung mit Benutzerprofilen, die jeweils einer Benutzeridentität zugeordnet sind, betreibbar ist und
die Personenortungs-/erkennungsvorrichtung (4) angepasst ist, Informationen über eine Benutzeridentität der Person einzuholen und zu verarbeiten, und
die Anzeigevorrichtung (2) und/oder Vorrichtung (1) zur extrakorporalen Blutbehandlung dazu angepasst ist/sind, in Abhängigkeit von der Benutzeridentität der Person gemäß dem zugehörigen Benutzerprofil bedienbar zu sein.

6. Vorrichtung (1) zur extrakorporalen Blutbehandlung nach Anspruch 1 bis 5, **dadurch gekennzeichnet, dass** die Personenortungs-/erkennungsvorrichtung (4) eine Kamera aufweist.

7. Vorrichtung (1) zur extrakorporalen Blutbehandlung nach Anspruch 1 bis 6, **dadurch gekennzeichnet, dass** die Personenortungs-/erkennungsvorrichtung (4) eine Gesichtserkennungsvorrichtung (10) aufweist.

8. Vorrichtung (1) zur extrakorporalen Blutbehandlung nach Anspruch 1 bis 7, **dadurch gekennzeichnet, dass** die Vorrichtung (1) zur extrakorporalen Blutbehandlung mit einer Vorrichtung für einen akustischen Alarm ausgestattet ist, und
die Personenortungs-/erkennungsvorrichtung (4) und die Vorrichtung für einen akustischen Alarm über die Steuereinheit (9) in Informationsaustauschkontakt zueinander stehen und
die Vorrichtung für einen akustischen Alarm angepasst ist, durch Signale der Personenortungs-/erkennungsvorrichtung (4) bedienbar zu sein.

9. Vorrichtung (1) zur extrakorporalen Blutbehandlung nach Anspruch 1 bis 8, **dadurch gekennzeichnet, dass** die Anzeigevorrichtung ein Display (3) mit einer konvex gebogenen, dem Krümmungsmittelpunkt abgewandten Displayfläche aufweist.

10. Vorrichtung (1) zur extrakorporalen Blutbehandlung nach Anspruch 1 bis 9, **dadurch gekennzeichnet, dass** die Anzeigevorrichtung (2) dazu angepasst ist, im Falle eines Alarms eine Kritikalitätsbewertung anzuzeigen.

11. Netzwerk aus mehreren Vorrichtungen zur extrakorporalen Blutbehandlung nach Anspruch 1 bis 9, **dadurch gekennzeichnet, dass** die Anzeigevorrichtungen der Vorrichtungen zur extrakorporalen Blutbehandlung dazu angepasst sind, im Fall von Alarmen an mehreren Geräten die auf den Displayflächen der Anzeigevorrichtungen darzustellenden Informationen gemäß der Priorität der Alarme darstellen.

## Claims

1. An apparatus (1) for extracorporeal blood treatment comprising a control unit (9) and a display device (2), and with a person locating/identifying device (4), wherein
the person locating/identifying device (4) is provided and adapted to obtain and to process information at least about the position of a person being located in a range detectable by the person locating/identifying device (4), and
the control unit (9) receives the information obtained and processed by the person locating/identifying device (4),
the person locating/identifying device (4) and the display device (2) are in contact of information exchange with each other via the control unit (9), **characterized in that**
the control unit (9) calculates a current relative position of the person to the apparatus (1) from the received information,
the display device (2) is provided with an external, circumferential display (3, 7) which is visible from all positions inside the range detectable by the person locating/identifying device (4), and
the display device (2) is provided and adapted to orient the display direction of the information to be displayed in response to at least the relative position of the person to the apparatus (1) for extracorporeal blood treatment automatically in the direction of the position of the person so that the information to be displayed is visible from the position of the person.

2. The apparatus (1) for extracorporeal blood treatment according to claim 1, **characterized in that**
the person locating/identifying device (4) is provided and adapted to obtain and to process information about gestures of the person, and
the display device (2) is adapted to be operable by the information about the gestures of the person.

3. The apparatus (1) for extracorporeal blood treatment according to claim 1 or 2, **characterized in that**
the person locating/identifying device (4) is adapted to obtain and to process information about gestures of the person and
the apparatus (1) for extracorporeal blood treatment is adapted to be operable by the information about the gestures of the person.

4. The apparatus (1) for extracorporeal blood treatment according to claim 1 to 3, **characterized in that**
the apparatus (1) for extracorporeal blood treatment can be operated with user profiles each being assigned to a user identity, and
the person locating/identifying device (4) is adapted to obtain and to process information about a user identity of the person, and
the display device (2) is adapted to display information in response to the user identity of the person according to the pertinent user profile on a display (3, 7).

5. The apparatus (1) for extracorporeal blood treatment according to claim 1 to 4, **characterized in that**
the apparatus (1) for extracorporeal blood treatment can be operated with user profiles each being assigned to a user identity, and
the person locating/identifying device (4) is adapted to obtain and to process information about a user identity of the person, and
the display device (2) and/or the apparatus (1) for extracorporeal blood treatment is/are adapted to be operable in response to the user identity of the person according to the pertinent user profile.

6. The apparatus (1) for extracorporeal blood treatment according to claim 1 to 5, **characterized in that** the person locating/identifying device (4) includes a camera.

7. The apparatus (1) for extracorporeal blood treatment according to claim 1 to 6, **characterized in that** the person locating/identifying device (4) includes a face recognition device (10).

8. The apparatus (1) for extracorporeal blood treatment according to claim 1 to 7, **characterized in that** the apparatus (1) for extracorporeal blood treatment is equipped with a device for acoustic alarm and
the person locating/identifying device (4) and the device for acoustic alarm are in contact of information exchange with each other via the control unit (9) and
the device for acoustic alarm is adapted to be operable by signals of the person locating/identifying device (4).

9. The apparatus (1) for extracorporeal blood treatment according to claim 1 to 8, **characterized in that** the display device (2) includes a display (3) having a convexly curved display surface turned away from the center of curvature.

10. The apparatus (1) for extracorporeal blood treatment according to claim 1 to 9, **characterized in that** the display device (2) is adapted to display a criticality assessment in the event of alarm.

11. A network of a plurality of apparatuses for extracorporeal blood treatment according to claim 1 to 9, **characterized in that** the display devices of the apparatuses for extracorporeal blood treatment are adapted to display, in the case of alarm on a plurality of apparatuses, the information to be displayed on the display surfaces of the display devices according to the priority of the alarms.

## Revendications

1. Dispositif (1) pour le traitement sanguin extracorporel avec une unité de commande (9), un dispositif d'affichage (2) et un dispositif de localisation/reconnaissance de personnes (4), dans lequel
le dispositif de localisation/reconnaissance de personnes (4) est prévu et adapté afin d'obtenir et de traiter des informations au moins sur la position d'une personne, qui se trouve dans une zone qui peut être détectée par le dispositif de localisation/reconnaissance de personnes (4),
l'unité de commande (9) reçoit les informations obtenues et traitées par le dispositif de localisation/reconnaissance de personnes et
le dispositif de localisation/reconnaissance de personnes (4) et le dispositif d'affichage (2) sont en contact d'échange d'informations l'un avec l'autre par le biais de l'unité de commande (9), **caractérisé en ce que**
l'unité de commande (9) calcule à partir des informations reçues une position relative actuelle de la personne par rapport au dispositif (1),
le dispositif d'affichage (2) est équipé d'un écran périphérique extérieur (3 ; 7) qui est visible depuis l'ensemble des positions à l'intérieur de la zone détectable par le dispositif de localisation/reconnaissance de personnes (4), et
le dispositif d'affichage (2) est prévu et adapté afin d'orienter le sens d'affichage des informations à afficher en fonction au moins de la position relative de la personne par rapport au dispositif (1) pour le traitement sanguin extracorporel automatiquement sur la position de la personne de sorte que les informations à afficher puissent être triées depuis la position de la personne.

2. Dispositif (1) pour le traitement sanguin extracorporel selon la revendication 1, **caractérisé en ce que**
le dispositif de localisation/reconnaissance de personnes (4) est prévu et adapté afin d'obtenir et de traiter des informations sur des gestes de la personne, et
le dispositif d'affichage (2) est adapté afin d'être commandable par les informations sur les gestes de la personne.

3. Dispositif (1) pour le traitement sanguin extracorporel selon la revendication 1 ou 2, **caractérisé en ce que**
le dispositif de localisation/reconnaissance de personnes (4) est adapté afin d'obtenir et de traiter des informations sur des gestes de la personne, et
le dispositif (1) pour le traitement sanguin extracorporel est adapté afin d'être commandable par les informations sur les gestes de la personne.

4. Dispositif (1) pour le traitement sanguin extracorporel selon la revendication 1 à 3, **caractérisé en ce que**
le dispositif (1) pour le traitement sanguin extracorporel peut être utilisé avec des profils utilisateur qui sont associés respectivement à une identité utilisateur, et
le dispositif de localisation/reconnaissance de personnes (4) est adapté afin d'obtenir et de traiter des informations sur une identité utilisateur de la personne, et
le dispositif d'affichage (2) est adapté afin de représenter sur un écran (3 ; 7) des informations en fonction de l'identité utilisateur de la personne selon le profil utilisateur afférent.

5. Dispositif (1) pour le traitement sanguin extracorporel selon la revendication 1 à 4, **caractérisé en ce que**
le dispositif (1) pour le traitement sanguin extracorporel peut être utilisé avec des profils utilisateur qui sont associés respectivement à une identité utilisateur et
le dispositif de localisation/reconnaissance de personnes (4) est adapté afin d'obtenir et de traiter des informations sur une identité utilisateur de la personne, et
le dispositif d'affichage (2) et/ou dispositif (1) pour le traitement sanguin extracorporel est/sont adapté(s) afin d'être commandable en fonction de l'identité utilisateur de la personne selon le profil utilisateur afférent.

6. Dispositif (1) pour le traitement sanguin extracorporel selon la revendication 1 à 5, **caractérisé en ce que** le dispositif de localisation/reconnaissance de personnes (4) présente une caméra.

7. Dispositif (1) pour le traitement sanguin extracorporel selon la revendication 1 à 6, **caractérisé en ce que** le dispositif de localisation/reconnaissance de personnes (4) présente un dispositif de reconnaissance de visage (10).

8. Dispositif (1) pour le traitement sanguin extracorporel selon la revendication 1 à 7, **caractérisé en ce que** le dispositif (7) pour le traitement sanguin extracorporel est équipé d'un dispositif pour une alarme acoustique, et
le dispositif de localisation/reconnaissance de personnes (4) et le dispositif pour une alarme acoustique sont en contact d'échange d'information l'un avec l'autre par le biais de l'unité de commande (9) et
le dispositif pour une alarme acoustique est adapté afin d'être commandable par des signaux du dispositif de localisation/reconnaissance de personnes (4).

9. Dispositif (1) pour le traitement sanguin extracorporel selon la revendication 1 à 8, **caractérisé en ce que** le dispositif d'affichage présente un écran (3) avec une surface d'écran pliée de manière convexe, éloignée du point médian de courbure.

10. Dispositif (1) pour le traitement sanguin extracorporel selon la revendication 1 à 9, **caractérisé en ce que** le dispositif d'affichage (2) est adapté afin d'afficher dans le cas d'une alarme une évaluation de criticité.

11. Réseau composé de plusieurs dispositifs pour le traitement sanguin extracorporel selon la revendication 1 à 9, **caractérisé en ce que** les dispositifs d'affichage des dispositifs pour le traitement sanguin extracorporel sont adaptés afin de représenter dans le cas d'alarmes au niveau de plusieurs appareils les informations à représenter sur les surfaces d'écran des dispositifs d'affichage selon la priorité des alarmes.
